# EUROPEAN PATENT SPECIFICATION

(11) **EP 2 015 777 B1**
(45) Date of publication and mention of the grant of the patent: **18.08.2010**
(21) Application number: 07783060.2
(22) Date of filing: 02.05.2007
(51) Int. Cl.: A61K 45/00

(54) **USE OF ORGANIC COMPOUNDS**
VERWENDUNG VON ORGANISCHEN VERBINDUNGEN
UTILISATION DE COMPOSÉS ORGANIQUES

(30) Priority: 03.05.2006 US 797228 P
(43) Date of publication of application: 21.01.2009
(73) Proprietor: Novartis AG, 4056 Basel (CH)
(72) Inventor: RANGWALA, Shamina M., Brookline, Massachusetts 02445 (US); STEVENSON, Susan C., Ashland, Massachusetts 01721 (US); WU, Zhidan, Boston, Massachusetts 02120 (US)
(74) Representative: Vögeli-Lange, Regina
(86) International application number: PCT/US2007/067971
(87) International publication number: WO 2007/131005

(56) References cited:
- WO-A-2006/130503
- US-A1- 2005 074 765
- ZUERCHER ET AL: "Identification and structure-activity relationship of phenolic acyl hydrazones as selective agonists for the estrogen related orphan nuclear receptors ERR beta and ERR gamma" J.MED.CHEM, vol. 48, 2005, - 2005 pages 3107-3109, XP002451477 cited in the application
- YU ET AL: "Identificatoin of an agonist ligand for estrogen related receptors ERR beta/gamma" NIOOIRGANIC AND MEDICINAL CHEMISTRY LETTERS, vol. 15, 2005, - 2005 pages 1311-1313, XP002451478 cited in the application

## Description

The invention relates to the use of an Estrogen-related receptor gamma (ERRγ) agonist or a pharmaceutically acceptable salt thereof for the treatment of a disease or condition selected from diabetes preferably type 2 diabetes, insulin resistance, metabolic disease/metabolic syndrome, dyslipidemia, obesity, overweight, Neurodegenerative diseases such as Parkinson's disease, Alzheimer's disease, Huntington's disease, or improvement of exercise endurance capacity, by administering to a said animal in need of such treatment an effective dose of at least one Estrogen-related receptor gamma agonist or a pharmaceutically acceptable salt thereof.

The estrogen-related receptors (ERR) comprise 3 members, ERRα, ERRβ, and ERRγ, that form a subfamily of orphan nuclear receptors for which natural ligands have yet to be identified. The ERRs share significant amino acid homology with the estrogen receptor (ER) within their DNA binding domains (DBD) and ligand binding domains (LBD), but do not respond to estradiol. Whereas ERs are ligand-activated receptors, ERRs can activate gene transcription in a constitutive manner and their activation ability may be determined by the presence of transcriptional coactivators. Structural studies confirm this finding by demonstrating that the ERRγ LBD can adopt a transcriptionally active conformation and interact with the steroid receptor coactivator 1 (SRC-1) in the absence of any ligand.

Within its DBD, ERRγ shares 93% and 99% amino acid identity with ERRα and ERRβ, respectively. The ERRs are less conserved within their LBDs (61% and 77% amino acid identity with ERRγ vs. ERRα and ERRβ, respectively;). The coactivator, peroxisome proliferator-activated receptor-γ coactivator-1α (PGC-1α) has been reported to be an endogenous protein ligand for ERRα and ERRγ (Hentschke M, et al. (2002) - PGC-1 and PERC, coactivators of the estrogen receptor-related receptor γ. Biochem Biophys Res Commun; 299:872-9).

Evidence indicates that ERRα and PGC-1α function in concert to regulate the expression of genes involved in mitochondrial biogenesis and oxidative phosphorylation. ERRα, ERRγ, and PGC-1α are coexpressed in tissues which utilize mitochondrial fatty acid oxidation as their primary energy source such as skeletal muscle, heart, and kidney (Hong H, Yang L, Stallcup MR (1999) - Hormone-independent transcriptional activation and coactivator binding by novel orphan nuclear receptor ERR3. J Biol Chem; 274:22618-26), and recently it was shown that ERRγ is a potent activator for ERRα gene expression, which is enhanced by PGC-1α (Liu D, Zhang Z, Teng CT (2005) - Estrogen-related receptor-γ and peroxisome proliferator-activated receptor-γ coactivator-1α regulate estrogen-related receptor-α gene expression via a conserved multi-hormone response element. J Mol Endocrinol; 34:473-87). The ERRγ LBD proteins were shown to be active in the absence of agonists but activity was potentiated with the addition of known agonist compounds, GSK4716 (Glaxo-Smith-Kline; NVP-AJQ710) and GSK9089 (Glaxo-Smith-Kline; NVP-LCN446) (Zuercher WJ, et al. (2005) Identification and structure-activity relationship of phenolic acyl hydrazones as selective agonists for the estrogen-related orphan nuclear receptors ERRβ and ERRγ. J Med Chem; 48:3107-9).

The US patent application US 2005/0096384 speculated on the potential use of ligands targeting the ERR receptors. This application speculated particularly on the use of ERR antagonists for the treatment of several indications.

Pursuing research in this field, the applicant has surprisingly discovered that contrarily to the teaching of US 2005/0096384, only stimulation of ERRγ activation by the use of ERRγ agonists results in enhanced mitochondriogenesis.

The applicant has further discovered that stimulation of ERRγ activation with a ERRγ agonist, results in unexpected good effects for the treatment of a disease selected from diabetes, type 2 diabetes, insulin resistance, metabolic disease/metabolic syndrome, dyslipidemia, obesity/overweight, neurodegenerative diseases, or for the improvement of exercise endurance capacity.

To facilitate the profiling of ERRγ agonists, the applicant has developed a fluorescence resonance energy transfer (FRET) assay (described hereinafter) that measures changes in the interaction between the LBD and a coactivator peptide in response to ligand binding. The assay (Zuercher, et al. 2005, "Identification and structure-activity relationship of phenolic acyl hydrazones as selective agonists for the estrogen-related orphan nuclear receptors ERRbeta and ERRgamma."; J Med Chem. 2005 May 5;48(9):3107-9.) has been validated using two known small agonists of ERRγ i.e. GSK4716 (Glaxo-Smith-Kline; AJQ710) and GSK9089 (Glaxo-Smith-Kline; LCN446).

The ERRγ LBD proteins are shown to be active in the absence of agonists but activity is potentiated with the addition of known agonist compounds, GSK4716 (Glaxo-Smith-Kline) and GSK9089 (Glaxo-Smith-Kline) (Zuercher, et al. 2005).

The term "ERRγ agonist" is intended to indicate a molecule that exhibits activation of the activity of ERRγ, such as from 1-100% activation, and specially preserves the action of substrate molecules. Preferably the ERRγ agonist exhibits an EC₅₀ comprised between 0.001 and 10 micro Molar (or between 0.001 and 1 micro Molar) in an ERRγ FRET assay (ability of the ERRγ agonist to induce an increased FRET response). Preferably the ERRγ agonist exhibits an EC₅₀ comprised between 0.001 and 10 micro Molar (or between 0.001 and 1 micro Molar) in the below described assays. Preferably the "agonist of ERRγ" refers to a molecule which when bound to the LBD sequence of ERRγ, increases the amount of, or prolongs the duration of, or enhances the activity of ERRγ. Agonists can include polypeptides, nucleic acids, carbohydrates, lipids, or any derivatives thereof, or any other molecules.

ERRγ agonist can be isolated by screening compounds as described by Zuercher, et al. 2005, or by Coward, P et al. - 2001, ("4-Hydroxytamoxifen binds to and deactivates the estrogen-related receptor gamma." Proc Natl Acad Sci USA. 2001 Jul 17;98(15):8880-4. Epub 2001 Jul 10), and Zhou G. et al. - 1998, (" Characterization by Fluorescence Resonance Energy Transfer"; Mol. Endocrin. 1998, 12, 1594-1604), which assays are incorporated in the present application by reference. Preferably the ERRγ agonists can be isolated by screening compounds as described in the below described assay (see experimental part) developed by the applicant.

In the present context "ERRγ agonist is also intended to comprise active metabolites and prodrugs thereof, such as active metabolites and prodrugs of ERRγ agonists. A "metabolite" is an active derivative of a ERRγ agonist produced when the ERRγ agonist is metabolised. A "prodrug" is a compound that is either metabolised to a ERRγ agonist or is metabolised to the same metabolite(s) as a ERRγ agonist.

ERRγ agonists are known in the art. For example, ERRγ agonists are in each case generically and specifically disclosed e.g. in Zuercher, et al. 2005.

Preferred ERRγ agonists are GSK4716 or GSK9089.

GSK4716 is known as 4-hydroxy-N'-(4-isopropylphenylmethylidene)-benzohydrazide of the formula 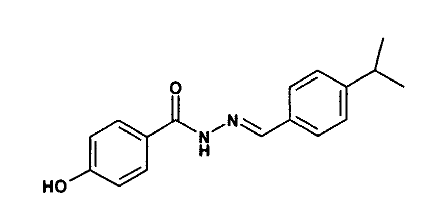 and is described by Zuercher, et al. 2005.

GSK9089 (or DY131) is known as N'-(4-(diethylamino)phenylmethylidene)-4-hydroxybenzohydrazide of the formula 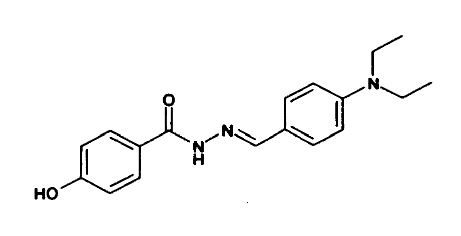 and has a CAS Registry number of 095167-41-2. The compound GSK9089 and its activity on ERRγ is also described by D. D. Yu and col. ( Identification of an agonist ligand for estrogen-related receptors ERRβ/γ Bioorg. Med. Chem. Lett. 15 (2005) 1311-1313) and by Zuercher, et al. 2005.

Especially preferred are orally active ERRγ agonists inhibitors.

Any of the substances disclosed in the above mentioned patent documents, hereby included by reference, are considered potentially useful as ERRγ agonists to be used in carrying out the present invention.

ERRγ agonists to be used alone according to the present invention can be used in association with a carrier.

A carrier in the instant context is a tool (natural, synthetic, peptidic, non-peptidic) for example a protein which transports specific substances through the cell membrane in which it is embedded and into the cell. Different carriers (natural, synthetic, peptidic, non-peptidic) are required to transport different substances, as each one is designed to recognize only one substance, or group of similar substances.

Any means of detection known by the person skilled in the art can be used to detect the association of the ERRγ agonists with a carrier, for example, by labelling the carrier.

Most preferred are orally active ERRγ agonists and pharmaceutical salts thereof.

The active ingredients or pharmaceutically acceptable salts thereof according to the present invention may also be used in form of a solvate, such as a hydrate or including other solvents, used for crystallization.

It has now been surprisingly found that ERRγ agonists are useful for the prevention, delay of progression or the treatment of a disease or condition selected from diabetes preferably type 2 diabetes, insulin resistance, metabolic disease/metabolic syndrome, dyslipidemia, obesity, overweight.

The present invention thus concerns the use of a ERRγ agonist e.g. GSK4716 or GSK9089, or pharmaceutically acceptable salt thereof, for the manufacture of a medicament for the prevention, delay of progression or the treatment of a disease or condition selected from diabetes preferably type 2 diabetes, insulin resistance, metabolic disease/metabolic syndrome, dyslipidemia, obesity, overweight.

In a further embodiment, the present invention relates to a pharmaceutical composition comprising a therapeutically effective amount of a ERRγ agonist in combination with one or more pharmaceutically acceptable carriers for the treatment of a disease or condition selected from diabetes preferably type 2 diabetes, insulin resistance, metabolic disease/metabolic syndrome, dyslipidemia, obesity, overweight.

Most preferably, according to the present invention the treated diseases or damages are selected from type 2 diabetes, metabolic disease/metabolic syndrome, dyslipidemia.

In the present description, the term "treatment" includes both prophylactic or preventative treatment as well as curative or disease suppressive treatment, including treatment of patients at risk of contracting the disease or suspected to have contracted the disease as well as ill patients. This term further includes the treatment for the delay of progression of the disease.

The term "curative" as used herein means efficacy in treating ongoing diseases.

The term "prevention" means prophylactic administration of the combination to healthy patients to prevent the outbreak of the conditions mentioned herein. Moreover, the term "prevention" means prophylactic administration of such combination to patients being in a pre-stage of the conditions, to be treated.

The term "prophylactic" means the prevention of the onset or recurrence of diseases or damages.

The term "delay of progression" as used herein means administration of the active compound to patients being in a pre-stage or in an early phase of the disease to be treated, in which patients for example a pre-form of the corresponding disease is diagnosed or which patients are in a condition, e.g. during a medical treatment or a condition resulting from an accident, under which it is likely that a corresponding disease will develop.

The metabolic syndrome is a cluster of risk factors that increases greatly the risk of occurrence of a cardiovascular event: diabetes or prediabetes, abdominal obesity, changes in cholesterol and high blood pressure. While up to 80 per cent of the almost 200 million adults worldwide with diabetes will die of cardiovascular disease, people with metabolic syndrome are also at increased risk, being twice as likely to die from and three times as likely to have a heart attack or stroke compared to people without the syndrome. People with metabolic syndrome have a fivefold greater risk of developing type 2 diabetes (if not already present). It is the exact nature of the cluster which appears to bring additional risk over and above that which would be expected from each of the components (high triglycerides when measuring cholesterol, for example). For a person to be defined as having the metabolic syndrome, the definition requires they have central obesity, plus two of the following four additional factors: raised triglycerides, reduced HDL cholesterol, raised blood pressure, or raised fasting plasma glucose level. Gender and ethnicity are also factors taken into consideration in the definition of metabolic syndrome.

The term "diabetes" as used herein means Type 2 diabetes, Type 1 diabetes and latent autoimmune diabetes of adulthood (LADA), preferably diabetes is Type 2 diabetes.

Type 2 diabetes usually develops in people older than 40 or people who are overweight (obese). In general, the treatment of type 2 diabetes patients does not involve insulin therapy but modification of certain lifestyle aspects (e.g. exercise, weight loss, a strict diet) and sometimes oral antidiabetics is sufficient to control blood glucose levels. Type 2 diabetes (Adult-Onset Diabetes Mellitus) generally occurs when the body develops insulin resistance a result of genetic factors and obesity, and is typically diagnosed in adulthood. Insulin resistance causes hyperglycemia and, because of prolonged demand for insulin production, deterioration of the pancreatic beta cells. The combination of insulin resistance and decreased beta cell function ultimately causes type 2 diabetes.

Type 1 diabetes is frequently diagnosed in childhood, and is sometimes referred to as juvenile diabetes for that reason. Early diagnosis is important to prevent some of the more serious complications of diabetes, which include heart disease, blindness, high blood pressure, nerve damage, and kidney failure. However, although type 1 diabetes tends to Occur most frequently in young, lean individuals, usually before 30 years of age, older patients do also present this form of diabetes. This type of type 1 diabetes is usually referred to as latent autoimmune diabetes of adulthood (LADA). Like the more common juvenile type 1 diabetes, LADA is caused by immune-mediated destruction of the insulin-producing pancreatic beta cells. LADA is also known as slow-onset type 1 diabetes, late-onset autoimmune diabetes of adulthood, and type 1 diabetes. The main difference between juvenile type 1 diabetes and LADA is the age of diagnosis - generally thirty years or older. A Methods for the diagnosis of LADA are e.g. described in the patent application WO2005054512 A2. Thus, type 1 diabetes can be present at any age, and the factors that determine the age of clinical manifestation are not known.

Impaired Glucose Metabolism (IGM) is defined by blood glucose levels that are above the normal range but are not high enough to meet the diagnostic criteria for type 2 diabetes mellitus. The incidence of IGM varies from country to country, but usually occurs 2-3 times more frequently than overt diabetes. Until recently, individuals with IGM were felt to be pre-diabetics, but data from several epidemiologic studies argue that subjects with IGM are heterogeneous with respect to their risk of diabetes and their risk of cardiovascular morbidity and mortality. The data suggest that subjects with IGM, in particular IGT, do not always develop diabetes, but whether they are diabetic or not, they are, nonetheless, at high risk for cardiovascular morbidity and mortality.

Among subjects with IGM, about 58% have Impaired Glucose Tolerance (IGT), another 29% have Impaired Fasting Glucose (IFG), and 13% have both abnormalities (IFG/IGT). IGT is characterized by elevated postprandial (post-meal) hyperglycemia while IFG has been defined by the ADA (see Table below) on the basis of fasting glycemic values.

Overweight: Weight loss i.e. treating/preventing/delaying overweight, is desirable in the case of diabetes, obesity and overweight individuals. Weight loss i.e. treating/preventing/delaying overweight, can help to prevent many of these harmful consequences, particularly with respect to diabetes and cardiovascular disease (CVD). Weight loss may also reduce blood pressure in both overweight hypertensive and non-hypertensive individuals; serum triglycerides levels and increases the beneficial high-density lipoprotein (HDL)-form of cholesterol. Weight loss also generally reduces somewhat the total serum cholesterol and low-density lipoprotein (LDL)- cholesterol levels. Weight loss may also reduce blood glucose levels in overweight and obese persons.

Weight loss, and hypocaloric diets, are also a primary goals for the control of plasma glucose levels in the treatment of type 2 diabetes. Thus appetite control and weight loss are desirable for the treatment of type 2 diabetes. The term "treatment of overweight" covers e.g. body fat reduction or weight loss.

The term "weight loss" refers to loss of a portion of total body weight.

The term "body fat reduction" means loss of a portion of body fat.

The formula for Body Mass Index (BMI) is [Weight in pounds÷Height in inches÷Height in inches]×703. BMI cutpoints for human adults are one fixed number, regardless of age or sex, using the following guidelines: Overweight human adults individuals have a BMI of 25.0 to 29.9. Obese human adults have a BMI of 30.0 or more. Underweight adults have a BMI less of than 18.5. A normal body weight range for an adult is defined as a BMI between 18.5 and 25. BMI cutpoints for children under 16 are defined according to percentiles: Overweight is defined as a BMI for age greater than >85th percentile and obesity is defined as a BMI-forage>95th percentile. Underweight is a BMI-for-age<5th percentile. A normal body weight range for a child is defined as a BMI above the 5th percentile and below the 85 percentile. The invention also relates to a pharmaceutical composition comprising, as active ingredients a ERRγ agonist, in free form or in form of a pharmaceutically acceptable salt thereof.

Another aspect of the present invention is the use of a pharmaceutical composition comprising, as active ingredients a ERRγ agonist preferably GSK4716 or GSK9089, in free form or in form of a pharmaceutically acceptable salt thereof for the preparation of a pharmaceutical composition for the prevention, delay of progression or treatment diabetes preferably type 2 diabetes, insulin resistance, metabolic disease/metabolic syndrome, dyslipidemia, obesity, overweight.

The invention also relates to a method for the prevention, delay of progression or treatment diabetes preferably type 2 diabetes, insulin resistance, metabolic disease/metabolic syndrome, dyslipidemia, obesity, overweight, Neurodegenerative diseases such as Parkinson's disease, Alzheimer's disease, Huntington's disease, or improvement of exercise endurance capacity, comprising administering to a warm-blooded animal, including man, in need thereof jointly therapeutically effective amounts of a pharmaceutical composition comprising, as active ingredients a ERRγ agonist preferably GSK4716 or GSK9089, in free form or in form of a pharmaceutically acceptable salt thereof.

The pharmaceutical compositions according to the invention can be prepared in a manner known per se and are those suitable for enteral, such as oral or rectal, and parenteral administration to mammals (warm-blooded animals), including man, comprising a therapeutically effective amount of the pharmacologically active compound, alone or in combination with one or more pharmaceutically acceptable carriers, especially suitable for enteral or parenteral application.

These pharmaceutical preparations are for enteral, such as oral, and also rectal or parenteral, administration to homeotherms, with the preparations comprising the pharmacological active compound either alone or together with customary pharmaceutical auxiliary substances. For example, the pharmaceutical preparations consist of from about 0.1% to 90 %, preferably of from about 1 % to about 80 %, of the active compound. Pharmaceutical preparations for enteral or parenteral, and also for ocular, administration are, for example, in unit dose forms, such as coated tablets, tablets, capsules or suppositories and also ampoules. These are prepared in a manner that is known per se, for example using conventional mixing, granulation, coating, solubulizing or lyophilising processes. Thus, pharmaceutical preparations for oral use can be obtained by combining the active compound with solid excipients, if desired granulating a mixture which has been obtained, and, if required or necessary, processing the mixture or granulate into tablets or coated tablet cores after having added suitable auxiliary substances.

The dosage of the active compound can depend on a variety of factors, such as mode of administration, homeothermic species, age and/or individual condition.

Preferred patients for the uses or methods according to the present invention are patients or animals suffering from diabetes (preferably type 2 diabetes), IGM (preferably IGT), obesity or overweight, metabolic disease dyslipidemia, neurodegenerative disease or low exercise endurance capacity.

In a further preferred embodiment the present invention concerns the compositions, uses or methods according to the present invention for the prevention, or delay of progression, of diabetes (preferably type 2 diabetes), in a patient suffering from IGM (preferably IGT).

Therefore the invention also concerns;
- A method for the prevention or delay of progression of type 2 diabetes, comprising administering to a warm-blooded animal, including man, suffering from IGM, preferably IGT, a therapeutically effective amount of a ERRγ agonist.
- A pharmaceutical composition comprising a therapeutically effective amount of a ERRγ agonist in combination with one or more pharmaceutically acceptable carriers for the prevention, or delay of progression of type 2 diabetes, in a patient suffering from IGM, preferably IGT.
- The use of a ERRγ agonist or pharmaceutically acceptable salt thereof, for the manufacture of a medicament for the prevention, or delay of progression of type 2 diabetes, in a patient suffering from IGM, preferably IGT.

The invention also concerns any of the herein described pharmaceutical compositions, methods or uses, wherein the ERRγ agonist exhibits an EC₅₀ comprised between 0.001 and 10 micro Molar e.g. in an ERRγ FRET assay (ability of the ERRγ agonist to induce an increased FRET response) or in any of the herein described assays. Preferably the ERRγ agonist exhibits an EC₅₀ comprised between 0.001 and 1 micro Molar in the herein described assays (e.g. FRET assay of below example 1).

Preferred dosages, for those active ingredients of the pharmaceutical combination according to the present invention that are commercially available, are especially therapeutically effective commercially available dosages.

The dosage of the active compound can depend on a variety of factors, such as mode of administration, homeothermic species, age and/or individual condition.

The corresponding active ingredient or a pharmaceutically acceptable salt thereof may also be used in form of a hydrate or include other solvents used for crystallization.

For these indications, the exact dosage will of course vary depending upon the compound employed, mode of administration and treatment desired. The compound may be administered by any conventional route, non-oral or preferably orally.

Expected therapeutic results are obtained when administered at a daily dosage of from about 0.01 to 100mg/kg, more preferred doses ranged from 0.1 to 50mg/kg For the larger mammals, an indicated total daily dosage is in the range from about 0.01 to 100mg/kg of the compound, conveniently administered in divided doses 2 to 4 times a day in unit dosage form containing for example from about 10 to about 100 mg of the compound in sustained release form.

Another preferred daily oral dosage in humans is between 1 mg and 1 g preferably between 10 mg and 500 mg e.g. 10 mg, or between 10 mg and 200 mg.

Appropriate unit doses for oral administration contain for example about 10 to about 500 mg of the active ingredient i.e. ERRγ agonist. Appropriate doses for parenteral administration contain for example about 10 to about 500 mg or 10 to about 200 mg of the compound. The compounds may be administered in similar manner to known standards for uses in these utilities. The suitable daily dosage for a particular compound will depend on a number of factors such as its relative potency of activity. A person skilled in the pertinent art is fully enabled to determine the therapeutically effective dosage.

The compound of the invention may be administered in free base for or as a pharmaceutically acceptable acid addition or quaternary ammonium salt. Such salts may be prepared in conventional manner and exhibit the same order of activity as the free forms.

If these compounds have, for example, at least one basic center, they can form acid addition salts. Corresponding acid addition salts can also be formed having, if desired, an additionally present basic center. The compounds having an acid group (for example COOH) can also form salts with bases. For example, the compounds to be combined can be present as a sodium salt, as a maleate or as a dihydrochloride. The active ingredient or a pharmaceutically acceptable salt thereof may also be used in form of a hydrate or include other solvents used for crystallization.

The pharmaceutical compositions according to the invention can be prepared in a manner known per se and are those suitable for enteral, such as oral or rectal, and parenteral administration to mammals (warm-blooded animals), including man, comprising a therapeutically effective amount of the pharmacologically active compound, alone or in combination with one or more pharmaceutically acceptable carries, especially suitable for enteral or parenteral application.

The structure of the active agents identified by code nos., generic or trade names may be taken from the actual edition of the standard compendium "The Merck Index" or from databases, e.g. Patents International (e.g. IMS World Publications). The corresponding content thereof is hereby incorporated by reference. Any person skilled in the art is fully enabled to identify the active agents and, based on these references, likewise enabled to manufacture and test the pharmaceutical indications and properties in standard test models, both *in vitro* and *in vivo.*

The pharmacological activity may, for example, be demonstrated in a clinical study or in the test procedure as essentially described hereinafter in a manner known to the skilled person.

Preferred ERRγ agonist for the uses and methods of the present invention are GSK4716 or GSK9089 and optionally in any case pharmaceutical salts thereof.

### EXPERIMENTAL PART

The following examples are carried out with ERRγ agonists to show their claimed activity.

### EXAMPLES

The examples below are non-limiting and are merely representative of various aspects and features of the present invention.

### Example 1 - Screening assay to isolate ERRγ agonists

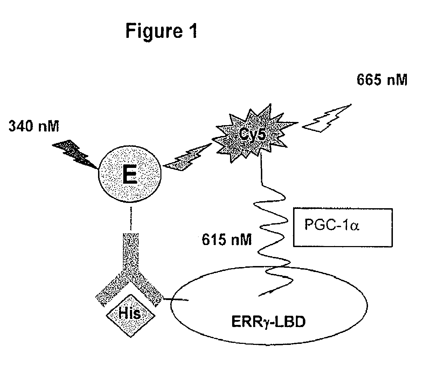

### FRET Assay to Detect Binding and compounds useful to carry out the present invention (see figure 1.1).

The FRET assay is designed to detect agonist-induced activation of ERRγ in the presence of the coactivator peptide PGC-1α. The following components were added in a final volume of 50 µL: (His)₆-hERRγ LBD or GST-hERRγ LBD, Europium-labeled anti-(HiS)₆ antibody or Europium-labeled anti-GST antibody and Cy5-labeled PGC-1α peptide (Cy5-RPCSELLKYLTT (SEQ ID NO. 4) with C-terminal acid, custom made and labeled at AnaSpec). Mix 1 contained antibody, hERRγ LBD in buffer in a volume of 19 µL and was added to 30 µL of Mix 2 which contained Cy5-peptide in buffer (1. 6xHis-ERRγ-LBD, 3.6mg/ml, MW 27KDa; 2. Cy5-PGC-1 peptide from AnaSpec; 3. Other buffer components from Sigma; 4. EU-antiHis₆-Ab from Perkin Elmer; and 5. Test compounds.). The assays were carried out in black 384-well plates and incubated at room temperature for 3hrs before FRET signals were measured using a Wallac Victor 2 (Perkin Elmer) plate reader. The ratio of emission signals (665 nm/615 nm) was used to determine the FRET assay response. In some cases, the FRET signal to background ratio was used and this is simply defined as the FRET ratio in the presence of protein or FRET ratio in the absence of protein. The test compounds were dissolved in DMSO at 10 mM and used as indicated.

### (a) Table 1 Reagent List

| **Reagent** | **Vendor** | **Catalog #** |
|---|---|---|
| Europium-labeled anti-(His)₆ antibody | Perkin Elmer | AD0110 |
| Europium-labeled anti-GST antibody | Perkin Elmer | AD0254 |
| Cy5-labeled PGC-1α peptide (Cy5-RPCSELLKYLTT (SEQ ID NO. 4); C-terminal acid of PGC-1α) | AnaSpec | Custom Order |
| Dithiothreitol | Sigma | D-0632 |
| Tris | Sigma | T-2194 |
| 384-well plates | Corning | 3654 |

The ability of agonist compounds to induce an increased FRET response between the Europium-labeled anti-(His)₆ antibody/(His)₆-hERRγ LBD complex and Cy5-RPCSELLKYLTT (SEQ ID NO. 4) was assessed by using two compounds described in the literature as ERRγ agonists, GSK4716 (Glaxo-Smith-Kline; AJQ710) and GSK9089 (Glaxo-Smith-Kline; LCN446. See Zuercher WJ, Gaillard S, Miller-Orband LA, et al. (2005), "Identification and structure-activity relationship of phenolic acyl hydrazones as selective agonists for the estrogen-related orphan nuclear receptors ERRβ and ERRγ," J Med Chem; 48:3107-9.). The compounds were solubilized in 10 mM DMSO and the concentration range of the titration was from 100 µM down to 2 nM. The final DMSO concentration was 2%. A DMSO control (no compound) at 2% was also prepared. All samples were made in duplicate. As shown in Figure 1.2, AJQ710 was able to induce up to a 60% increase in the FRET signal at the highest concentration tested. The FRET response is dose-dependent and an EC₅₀ of 2.1 µM was derived from the data. Similarly, LCN446 also induced a dose-dependent FRET response having a maximal response of 70% at saturating concentrations of compound with an EC₅₀ of 0.54 µM. Both EC₅₀'s were similar to the reported values of 1.3 µM and 0.13 µM for the AJQ71 0 and LCN446 equivalents, respectively.

### Example 2

### Characterization of AJQ710 on mitochondrial function in primary mouse myotubes

Primary mouse myoblasts from FVB mice were isolated and maintained as described in Bare et al., For experimentation, mouse myoblasts were grown to 80% confluence in F-10/Ham's media containing 20% fetal bovine serum, 1% Penicillin/Streptomycin and2.5 ng/mL bFGF (human recombinant). The cells were then plated to 700,000 cells per well in 6-well plates, and allowed to differentiate into myotubes for 36 to 48 hours in DMEM containing 5% equine serum and Penicillin/Streptomycin. The cells were treated with the ERRγ/β agonist AJQ710 for 24 hours. Assays performed included analysis of gene expression by real time quantitative PCR (RT-PCR), cytochrome c ELISA, citrate synthase assay, fatty acid oxidation assay, and a respiration assay. For the RT-PCR, cytochrome c ELISA and citrate synthase assay, myotubes were treated with AJQ710 at the following concentrations: 1 µM, 3 µM, 10 µM, and 30 µM. For the fatty acid oxidation assay, myotubes were treated with AJQ710 at the following concentrations: 10 µM, and 30 µM. For the respiration assay, myotubes were treated with AJQ710 at a concentration of 30 µM. Each dose was tested in trplicate, and a set of wells treated with an equivalent volume of DMSO used was included in every experimental readout for control purposes.

For the purpose of assessing gene expression by RT-PCR, RNA was isolated from cell lysates, and cDNA was subsequently synthesized from this RNA. For RNA isolation, cells were homogenized in TRlzol (Invitrogen, catalog number 15596-026, Carlsbad, CA), and total RNA was isolated following the manufacturer's instructions. RNA was quantified using the spectrophotometer.

Reverse transcription was performed using the BD Sprint^{™} PowerScript^{™} kit from BD Biosciences (catalog number 639562). Quantitative real time PCR for the following genes was performed using Assay-on-Demand primer probes from Applied Biosystems (See Post-text Table 8-1 for catalog numbers): B2M, ERRγ, ERRβ, ERRα, PGC-1α, PGC-1β, PPARα, PPARγ, PPARδ, COX-4, cytochrome c, UQCRB, CPT-1b, LCAD, MCAD, IDH3a, ATP-5b, UCP-2, and UCP-3.

Taqman real time quantitative PCR was performed and analyzed following the manufacturer's instructions (Applied Biosystems). Specifically, amplification was performed in triplicate, in a 10 µl reaction mixture. The reaction mixture included: 1X TaqMan® Universal PCR Master Mix (Applied Biosystems, catalog number 4304437), 1X of Assay-on-Demand primer probe, and 2 µl of cDNA sample. Gene expression was calculated by normalizing to total cDNA as measured by B2M endogenous control (Applied Biosystems, catalog number Mm00437762_ml). The samples were initially incubated for 2 min at 50°C for optimum uracyl-N-glycosylase activity. The PCR program started with a 95°C denaturing for 10 min, followed by 40 cycles of 95°C/15 sec and 60°C/1 min in a 384-well thermal cycler (Perkin-Elmer Applied Biosystems). Each amplification run contained "No Template" controls (buffer and primers only). Amplification data were collected by the 7700 Sequence Detector and analyzed using the Sequence Detection System software developed by Perkin-Elmer (Applied Biosystems). The fractional cycle number reflecting a positive PCR result is called the cycle threshold (Ct).

Average gene expression values were calculated for each group relative to B2M, using 2^{-ΔΔCt} (as described by Applied Biosystems, Foster City, CA) with the expression in the vehicle treated cells (0 µM) normalized to a value of 1. The data are expressed as mean ± SEM (n = 3 replicate wells). Statistical significance was measured using Student's t test.

The cytochrome c enzyme-linked immunosorbent assay (ELISA) was performed using the Rat/Mouse Cytochrome c Immunoassay kit from R&D Systems (catalog number MCTC0, R&D Systems, Minneapolis, MN) following the manufacturer's instructions.

The citrate synthase assay was performed following the procedure described in above in this application.

Respiration was measured in myotubes incubated with AJQ710 for 24 hrs according to the published method with modifications (St-Pierre, et al., JBC, 278(29): 26597-603 (2003)). Cells were washed once with phosphate-buffered saline (PBS) and treated for 5 min at 37°C with 2 ml of trypsin (Mediatech, catalog number 25-052-Cl). Without removing trypsin, 10 ml of DMEM plus 10% FBS was added into each well. The cells were transferred to a 15 ml tube and centrifuged for 5 min at 1000 rpm. The cells were washed once with the medium and pelleted before being resuspended in the assay buffer containing 25 mM glucose (Sigma, catalog number G-5400), 1 mM pyruvate (Invitrogen, catalog number 11360-070) and 2% bovine serum albumin (BSA) (MP Biomedicals, catalog number 103703) in D-PBS (Invitrogen, catalog number 14040-133). The cell suspension was diluted to 1x10⁶ cells per ml in the assay buffer and kept at 37°C until used. Oxygen consumption was measured with a Clark electrode according to the instructions provided by Hansatech (Norfolk, UK). One half of the cell suspension was used for each measurement. The concentrations of oligomycin (MP Biomedicals, catalog number 151786) and FCCP (4-(trifluoromethoxy) carbonyl cyanide phenylhydrazone) (Sigma, catalog number C2920) were 2 µg/ml and 2 to 5 µM, respectively. The experiments were performed in triplicate. The rate of respiration was measured by calculating the slope of of the flux of oxygen consumption using the software provided by the manufacturer.

Data manipulations and graph generation were performed using Microsoft Excel and GraphPad Prism 4 software. Data are presented as mean ± SEM. In all experiments, each dose was tested in triplicate. The only exception to this were the respiration measurements where the experiment was repeated three times. Statistical analysis was preformed using a two-tailed Student's t test.

### Results

We examined the expression of various markers of mitochondrial gene expression in differentiated mouse myotubes treated with AJQ710 at the following concentrations: 30 µM, 10 µM, 3 µM and 1 µM. Stock solutions were prepared such that a constant volume of drug solution was added to each well of cells. All effects were compared to that vehicle (DMSO) alone. Mitochondrial pathways/genes examined included oxidative phosphorylation, fatty acid oxidation, Krebs cycle, ATP synthase, and uncoupling proteins. Additionally, expression of transcriptional regulators functionally related to ERRγ were examined. For almost all of the genes examined, we found a dose-dependent increase in gene expression following treatment with AJQ710. All gene expression results are from 24-hour AJQ710 treatment.

Genes of the oxidative phosphorylation demonstrated an elevated expression with increasing concentrations of AJQ710. COX-4 expression was increased by 2-fold, cytochrome c by 3.7-fold (i.e. from 1.0 Gene/B2M without treatment with AJQ710 to 3.7 Gene/B2M when treated with AJQ710 at a concentration of 30 µM), and UQCRB by 2-fold when treated with AJQ710 at a concentration of 30 µM.

Similarly, three genes examined that are associated with fatty acid oxidation, were shown to be upregulated in a dose-dependent manner following 24-hour treatment with AJQ710. CPT-1 b expression was increased by over 3-fold (i.e. from 1.0 Gene/B2M without treatment to 3.0 Gene/B2M when treated with AJQ710 at a concentration of 30 µM), LCAD by 2.8-fold, and MCAD by 1.6-fold following 30 µM treatment of AJQ710 .

Two other mitochondrial genes examined, IDH3α, a component of the Krebs cycle, and ATP-5b, an ATP-synthesizing enzyme, demonstrated a dose-dependent induction in expression with increasing doses of AJQ710. IDH3α expression was increased by over 2-fold (i.e. from 1.0 Gene/B2M without treatment with AJQ710 to 2.0 Gene/B2M when treated with AJQ710 at a concentration of 30 µM), and ATP-5b was increased by 1.6-fold after 24 hour treatment with 30 µM of AJQ710.

We measured mRNA expression for the uncoupling proteins UCP-2 and UCP-3. UCP-2 showed marginal upregulation in expression with all doses of AJQ710, with its highest expression, 1.5-fold, following 10 µM treatment. UCP-3 showed a dose-dependent increase in expression, with an increase of over 3-fold i.e. from 1.0 Gene/B2M without treatment with AJQ710 to 3.0 Gene/B2M following 30 µM treatment of AJQ710.

We measured the expression of the ERR family in response to treatment of mytoubes with agonist. AJQ710 purportedly activates both ERRγ and ERRβ, but does not activate ERRα and the ERs (Zuercher, et al., J. Med. Chem., 48(9): 3107-9 (2005)). The expression of ERRα was increased to a greater extent than ERRβ or ERRγ following treatment with AJQ710. ERRγ was elevated to 1.4-fold expression, ERRβ showed a 1.5-fold increase in expression, and ERRα showed a 3.7-fold increase in expression with 30 µM treatment of AJQ710.

The co-activators PGC-1α and PGC-1β were also examined for gene expression. The expression of these transcripts showed a similar dose-dependent increase in expression, with PGC-1α elevated to 1.9-fold expression (i.e. from 1.0 Gene/B2M without treatment with AJQ710 to 1.9 Gene/B2M when treated with AJQ710 at a concentration of 30 µM) and PGC-1β elevated to 1.8-fold.

We further examined gene expression for the PPARs, a family of nuclear receptors that play an important role in lipid metabolism. PPARα and PPARδ showed no change in expression following AJQ710 treatment. PPARγ showed a dose-dependent increase in expression with a maximal 2.2-fold expression (i.e. from 1.0 Gene/B2M without treatment with AJQ710 to 2.2 Gene/B2M following 30 µM AJQ710 treatment.

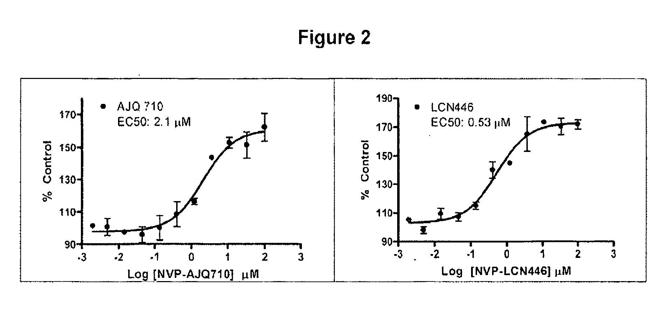

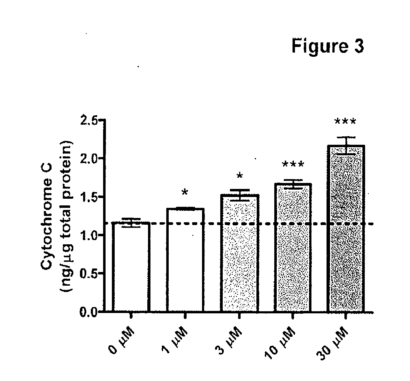

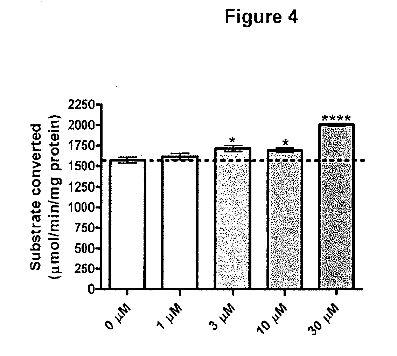

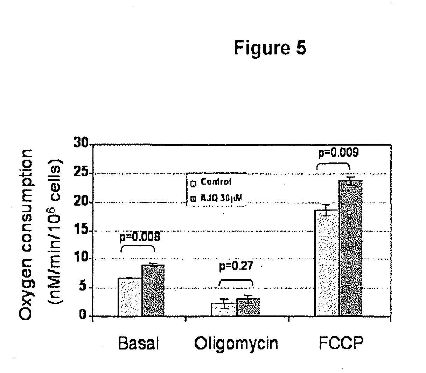

To assess mitochondrial activity at the protein level, a cytochrome c ELISA was performed. Cytochrome c is a critical element of the electron transport chain, and quantities of the protein serve as a biomarker for mitochondrial number and oxidative phosphorylation activity. We observed a dose-dependent increase of cytochrome c with compound treatment, with an increase of 88% when myotubes were treated with 30 µM AJQ710 for 24 hours (Figure 2.1).

To measure mitochondrial activity, citrate synthase activity was determined. This enzyme is often used as an indicator of mitochondrial content or activity in human muscle (Kelley, et al., Diabetes, 51 (10): 2944-50 (2002)). Citrate synthase catalyzes the initial step of the Krebs cycle, which supplies substrate for oxidative phosphorylation. Myotubes treated with 30 µM AJQ710 for 24 hours showed a 28% increase in citrate synthase activity, while 10 µM and 3 µM treatment showed an increase of 8% and 9% respectively (Figure 2.2).

We further investigated whether induction of mitochondrial gene expression has an impact on oxidative phosphorylation. Cellular respiration was measured in mouse primary myotubes treated with AJQ710 for 24 hr. Consistent with the induction of expression of the respiratory chain components, basal respiration was increased by 37% in AJQ710-treated muscle cells, compared with the vehicle treated controls (Figure 2.3). Oligomycin-insensitive respiration, the proton leak, was increased by 36%, although this increase was not statistically significant. In the presence of FCCP, respiration rate was increased by 32% by AJQ71 0, as compared to the vehicle-treated control cells (Figure 2.3). These results indicate that an ERRγ agonist can increase mitochondrial oxidative capacity through coupled respiration.

The improvement in mitochondrial function which can be observed from the above assays would indicated therapeutic benefits in the herein claimed indications.

### BRIEF DESCRIPTION OF FIGURES

Figure 1.1 shows the diagram of FRET assay configuration.
Figure 1.2 shows the agonist-induced FRET response.
Figure 2.1 shows protein expression levels of cytochrome c in mouse myotubes treated with NVP-AJQ710-NX-2.
Figure 2.2 shows citrate synthase activity in mouse myotubes treated with NVP-AJQ710-NX-2.
Figure 2.3 shows measurement of cellular respiration in primary mouse myotubes treated with NVP-AJQ710-NX-2.

### Example 3 - Body weight/obesity

Diet-induced obese mice are used for the study at 21-23 weeks of age. On the first day of the study, animals are fasted at 7:30 a.m. Body weight measurement and basal blood sample collection are conducted at 10:30 a.m. Animals were assigned into two groups (n = 10/group) with the plasma glucose values and body weights matched between the two groups. The animals are dosed orally with vehicle (water) or compound at 30 mg/kg at a dose volume of 5 ml/kg. Daily dose of vehicle or the compound is administered at the same time each day for a total of 28 days. Daily body weight and food intake measurements are taken during the study. Compounds decrease body weight in the treated animals, but do not affect food intake. Fat and lean mass analyses are performed at weekly intervals during the 28 day period using the EchoMRI Whole Body Composition Analyzer. Scans are performed using the appropriate size holders provided by the manufacturer. Animals that are administered the drug show a decrease in fat mass and a concomitant increase in lean mass. On day 24 of the study, animals are placed in the CLAMS system (Columbus Instruments, Columbus, OH) which measures the volume of oxygen consumed (VO₂) and volume of carbon dioxide produced (VCO₂) through air sampling in sealed chambers. The VO₂ is the measure of the overall oxidative capacity of the animals. The respiratory quotient (RQ) is calculated as the ratio of volume of CO₂ produced divided by volume of O₂ consumed and is a measure of substrate utilization. If the animals use fat as the major fuel source, the ratio is closer to 0.7, while if the fuel source is carbohydrate, the RQ is closer to 0.7. Animals treated with drug show a greater oxidative capacity as compared to the control animals. Due to a greater capacity to burn fat, treated animals exhibit lower RQ levels. On the last day of the study (day 28), animals are dosed with vehicle or compound at 10:30 a.m. Tail blood samples are taken at 12:30 p.m. Animals are then euthanized with carbon dioxide. Terminal blood samples are collected via cardiac puncture for blood chemistry analysis.

### Example 4 - Type 2 diabetes/Metabolic disease

Diet-induced obese mice are used for the study at 21-23 weeks of age. On the first day of the study, animals are fasted at 7:30 a.m. Body weight measurement and basal blood sample collection are conducted at 10:30 a.m. Plasma glucose values are then determined. Animals were assigned into two groups (n = 10/group) with the plasma glucose values and body weights matched between the two groups. At 12 pm, the animals are dosed orally with vehicle (water) or compound at 30 mg/kg at a dose volume of 5 ml/kg. At 1:00 p.m. a blood sample (at 0 min) is taken followed by an oral glucose tolerance test (OGTT) at 1 g/kg (20% glucose in water) at a dose volume of 5 ml/kg. Blood samples are collected at 30, 60 and 120 min following the glucose administration. The animals are refed after the OGTT. The animals are administered a daily dose of vehicle or the compound 12:00 p.m. each day for a total of 15 days. Daily body weight and food intake measurements are performed during the study. Two additional OGTTs are performed during the study on the days 7 and 14, following the protocol described above for the OGTT on day 1. Animals treated with drug show an improvement in glucose tolerance as compared to the control animals, as measured by the area under the curve during an OGTT. The magnitude of improvement in the OGTT increases in a time-dependent manner from day 7 to day 14. On the last day of the study (day 15), mice are fasted at 7:30 a.m. and dosed with vehicle or compound at 10:30 a.m. Tail blood samples are taken at 12:30 p.m. Animals are then euthanized with carbon dioxide. Terminal blood samples are collected via cardiac puncture for blood chemistry analysis.

### Blood collection and analyses

Blood samples are taken during the study via tail bleeding. Plasma glucose concentrations are determined using a glucose meter (Ascensia Elite, Bayer Corp., Mishawaka, IN). Blood samples were collected in tubes (Microvette CB300, Aktiengesellschaft & Co., Numbrecht, Germany) which contain lithium heparin to prevent blood clotting. Prior to each blood sample collection, 1 µl of 1:10 diluted protease inhibitor cocktail (Sigma, St. Louis, MO) is added to the sample tubes. After blood sample collection, the tubes are kept on ice before being centrifuged. The plasma portion of the blood samples is obtained by centrifugation at 10,000 x g for 10 min at 4 °C and then stored at -80 °C. Plasma insulin and glucagons levels are determined by Luminex assays using Mouse Endocrine Lincoplex kit (Linco Research, Inc., St. Charles, MO). Animals treated with drug i.e. ERRγ agonists show a lowering in plasma insulin levels as compared to the control animals. Plasma triglyceride, fatty acid and total cholesterol levels are determined using a fluorescent assay based on Amplex Red kit (Molecular Probes, Eugene, OR). Blood chemistry analysis is performed using an automated dry chemistry system (SPOTCHEM EZ Analyzer, Heska, Fort Collins, CO). Animals treated with the ERRγ agonists show a reduced body weight or improved lipid profile.

### Example 5 - Dyslipidemia

Diet-induced obese mice are used for the study at 21-23 weeks of age. On the first day of the study, animals are fasted at 7:30 a.m. Body weight measurement and basal blood sample collection are conducted at 10:30 a.m. Animals were assigned into two groups (n = 10/group) with the plasma glucose values and body weights matched between the two groups. The animals are dosed orally with vehicle (water) or compound at 30 mg/kg at a dose volume of 5 ml/kg. Daily dose of vehicle or the compound is administered at the same time each day for a total of 15 days. Daily body weight and food intake measurements are taken during the study. Fat and lean mass analyses are performed at twice during the 15 day period using the EchoMRI Whole Body Composition Analyzer. Scans are performed using the appropriate size holders provided by the manufacturer. On the last day of the study (day 15), mice are fasted at 7:30 a.m. and dosed with vehicle or compound at 10:30 a.m. Tail blood samples are taken at 12:30 p.m. Animals are then euthanized with carbon dioxide. Terminal blood samples are collected via cardiac puncture for blood chemistry analysis.

### Blood collection and analyses

Blood samples are taken during the study via tail bleeding. Plasma glucose concentrations are determined using a glucose meter (Ascensia Elite, Bayer Corp., Mishawaka, IN). Blood samples were collected in tubes (Microvette CB300, Aktiengesellschaft & Co., Numbrecht, Germany) which contain lithium heparin to prevent blood clotting. Prior to each blood sample collection, 1µl of 1:10 diluted protease inhibitor cocktail (Sigma, St. Louis, MO) is added to the sample tubes. After blood sample collection, the tubes are kept on ice before being centrifuged. The plasma portion of the blood samples is obtained by centrifugation at 10,000 x g for 10 min at 4 °C and then stored at -80 °C. Plasma insulin levels are determined by Luminex assays using Mouse Endocrine Linco*plex* kit (Linco Research, Inc., St. Charles, MO). Plasma triglyceride, fatty acid and total cholesterol levels are determined using a fluorescent assay based on Amplex Red kit (Molecular Probes, Eugene, OR). Animals treated with drug i.e. ERRγ agonists show a lowering in plasma triglyceride, free fatty acid and cholesterol levels as compared to the control animals. Blood chemistry analysis is performed using an automated dry chemistry system (SPOTCHEM EZ Analyzer, Heska, Fort Collins, CO).

### Example 6 - Exercise endurance capacity

To measure exercise capacity, age- and weight-matched C57BI6 mice are run on a motorized treadmill with a shock-plate incentive (Exer-6, Columbus Instruments, Columbus, OH). The mice are acclimatized to the treadmill on one day prior to the start of the experiment. On day one, the mice are exercised starting at 9 am for 2 hours. The speed of the treadmill is constant for the first hour, and increased by 2 meters/min every 15 minutes over the second hour. The incline of the treadmill is kept constant during the experiment. The capacity of the mice to run is assessed by measuring the time and speed of the treadmill until each mouse is exhausted and unable to stay on the treadmill. The total distance run and the work performed are calculated as measures of exercise capacity. Oxygen consumption during exercise, referred to as maximal oxidative capacity, is also measured to determine the tolerance of animals for exercise. The animals are then divided into two sets (n = 10 each), with exercise capacity matched between the two groups. At 12 pm, the animals are dosed orally with vehicle (water) or compound i.e. ERRγ agonists at 30 mg/kg at a dose volume of 5 ml/kg. Daily dose of vehicle or the compound is administered at 12:00 p.m. for a total of 28 days. Daily body weight and food intake measurements are taken during the study. On days 14 and 27 of the study, the animals are assessed for exercise capacity as described above. Animals treated with drug are able to run for a longer time and distance as compared to control animals. These animals also demonstrate an increase in maximal oxidative capacity with respect to the control subjects. The magnitude of the difference in these exercise capacity parameters between the treated and the control animals increases from day 14 to day 27 of the study. On day 28, the animals are dosed with vehicle or compound at 12 pm and tail blood samples are taken at 12:30 p.m. Animals are then euthanized with carbon dioxide, and tissues are collected for analysis of metabolites and gene expression. Terminal blood samples are collected via cardiac puncture for blood chemistry analysis. Blood samples were collected in tubes (Microvette CB300, Aktiengesellschaft & Co., Numbrecht, Germany) which contain lithium heparin to prevent blood clotting. Prior to each blood sample collection, 1µl of 1:10 diluted protease inhibitor cocktail (Sigma, St. Louis, MO) is added to the sample tubes. After blood sample collection, the tubes are kept on ice before being centrifuged. The plasma portion of the blood samples is obtained by centrifugation at 10,000 x g for 10 min at 4 °C and then stored at -80 °C. Plasma triglyceride, fatty acid and total cholesterol levels are determined using a fluorescent assay based on Amplex Red kit (Molecular Probes, Eugene, OR). Blood chemistry analysis is performed using an automated dry chemistry system (SPOTCHEM EZ Analyzer, Heska, Fort Collins, CO). Animals treated with drug i.e. ERRγ agonists show a lowering in plasma triglyceride, free acid and cholesterol levels as compared to the control animals.

## Claims

1. The use of an ERRγ agonist, in free form or in pharmaceutically acceptable salt form, for the manufacture of a medicament for the prevention, the delay of progression, or the treatment, of a disease or condition selected from the group, consisting of diabetes, insulin resistance, metabolic disease/metabolic syndrome, dyslipidemia, obesity and overweight.

2. The use according to claim 1, **characterized in that** the disease or condition is selected from the group, consisting of type 2 diabetes, metabolic disease/metabolic syndrome and dyslipidemia.

3. The use according to claim 1 or claim 2 for the manufacture of a medicament for the prevention, or the delay of progression, of type 2 diabetes in a patient suffering from IGM.

4. The use according to any one of claims 1 to 3, **characterized in that** the ERRγ agonist is selected from the group, consisting of GSK4716 and GSK9089, in each case in free form or in pharmaceutically acceptable salt form.

5. The use according to any one of claims 1 to 4, **characterized in that** the daily oral dosage of the ERRγ agonist is between 10 and 500 mg, or between 10 and 200 mg.

6. The use according to any one of claims 1 to 5, **characterized in that** the ERRγ agonist exhibits an EC₅₀ comprised between 0.001 and 10 micro Molar, or between 0.001 and 1 micro Molar.

7. The use according to any one of claims 1 to 6, **characterized in that** the ERRγ agonist exhibits an EC₅₀ comprised between 0.001 and 10 micro Molar, or between 0.001 and 1 micro Molar, in an ERRγ FRET assay.

## Patentansprüche

1. Verwendung eines ERRγ-Agonisten in freier Form oder in pharmazeutisch verträglicher Salzform für die Herstellung eines Medikaments zur Vorbeugung, Verzögerung des oder Behandlung einer Erkrankung oder eines Zustands, ausgewählt aus der Gruppe, betstehend aus Diabetes, Insulinresistenz, Stoffwechselerkrankung/Stoffwechselsyndrom, Dyslipidämie, Obesitas und Übergewicht.

2. Verwendung gemäß Anspruch 1, **dadurch gekennzeichnet, dass** die Erkrankung oder der Zustand ausgewählt ist aus der Gruppe, bestehend aus Typ-2-Diabetes, Stoffwechselerkrankung/Stoffwechselsyndrom und Dyslipidämie.

3. Verwendung gemäß Anspruch 1 oder Anspruch 2 für die Herstellung eines Medikaments zur Vorbeugung oder Verzögerung des Fortschreitend von Typ-2-Diabetes bei einem Patienten, der an IGM leidet.

4. Verwendung gemäß einem der Ansprüche 1 bis 3, **dadurch gekennzeichnet, dass** der ERRγ-Agonist ausgewählt ist aus der Gruppe, bestehend aus GSK4716 und GSK9089, jeweils in freier Form oder in pharmazeutisch verträglicher Salzform.

5. Verwendung gemäß einem der Ansprüche 1 bis 4, **dadurch gekennzeichnet, dass** die tägliche orale Dosierung des ERRγ-Agonisten zwischen 10 und 500 mg oder zwischen 10 und 200 mg beträgt.

6. Verwendung gemäß einem der Ansprüche 1 bis 5, **dadurch gekennzeichnet, dass** der EPRγ-Agcnisl eine EC₅₀ zwischen 0,001 und 10 mikromolar oder zwischen 0,001 und 1 mikromolar aufweist.

7. Verwendung gemäß einem der Ansprüche 1 bis 6, **dadurch gekennzeichnet, dass** der ERRγ-Agonist eine EC₅₀ zwischen 0,001 und 10 mikromolar oder zwischen 0,001 und 1 mikromolar bei einem ERRγ-FRET-Asssy aufweist

## Revendications

1. Utilisation d'un agoniste de l'ERRγ, sous forme libre ou sous forme d'un sel acceptable d'un point de vue pharmaceutique, pour la fabrication d'un médicament destiné à la prévention, au retardement de la progression ou au traitement d'une maladie ou d'un état pathologique choisi dans le groupe consistant en le diabète, la résistance à l'insuline, la maladie métabolique/le syndrome métabolique, la dyslipidémie, l'obésité est le surpoids.

2. Utilisation selon la revendication 1, **caractérisée en ce que** la maladie ou l'état pathologique est choisi dans le groupe consistant en le diabète de type 2, la maladie métobolique/le syndrome métabolique et la dyslipidémie.

3. Utilisation selon la revendication 1 ou 2 pour la fabrication d'un médicament destiné à la prévention ou au retardement de la progression d'un diabète de type 2 chez un patient souffrant, d'une altération du métabolisme du glucose.

4. Utilisation selon l'une quelconque des revendications 1 à 3, **caractérisée en ce que** l'agoniste de l'ERRγ est choisi dans le groupe consistant en le GSK4716 et le GSK9089, dans chaque cas sons forme libre ou sous forme d'un sel acceptable d'un point de vue pharmaceutique.

5. Utilisation selon l'une quelconque des revendications 1 à 4, **caractérisée en ce que** la posologie orale journalière de l'agoniste de l'ERRγ est comprise entre 10 et 500 mg, ou entre 10 et 200 mg,

6. Utilisation selon l'une quelconque des revendications 1 à 5, **caractérisée en ce que** l'agoniste de l'ERRγ présente une CE₅₀ comprise entre 0,001 et 10 micromolaire ou entre 0,001 et 1 micromolaire.

7. Utilisation selon l'une quelconque des revendications 1 à 6, **caractérisée en ce que** l'agoniste de l'ERRγ présente une CE₅₀ comprise entre 0,001 et 10 micromolaire ou entre 0,001 et 1 micromolaire dans un essai FRET sur ERRγ.
